# EUROPEAN PATENT SPECIFICATION

(11) **EP 4 385 403 B1**
(45) Date of publication and mention of the grant of the patent: **20.05.2026**
(21) Application number: 22216532.6
(22) Date of filing: 23.12.2022
(51) Int. Cl.: A61B 5/03, A61B 5/00

(54) **METHOD AND APPARATUS FOR MEASUREMENT AND MONITORING OF INTRAORBITAL PRESSURE WAVES**
VERFAHREN UND VORRICHTUNG ZUR MESSUNG UND ÜBERWACHUNG VON INTRAORBITALEN DRUCKWELLEN
PROCÉDÉ ET APPAREIL DE MESURE ET DE SURVEILLANCE D'ONDES DE PRESSION INTRA-ORBITALES

(30) Priority: 15.12.2022 US 202218081895
(43) Date of publication of application: 19.06.2024
(73) Proprietor: Kaunas University of Technology, 44249 Kaunas (LT)
(72) Inventor: RAGAUSKAS, Arminas, 51362 Kaunas (LT); PETKUS, Vytautas, 48300 Kaunas (LT)
(74) Representative: Sayettat, Julien Christian

(56) References cited:
- US-A1- 2009 287 084
- US-A1- 2018 279 877
- US-A1- 2018 296 182
- KOSKINEN LARS-OWE D ET AL: "Can intracranial pressure be measured non-invasively bedside using a two-depth Doppler-technique?", JOURNAL OF CLINICAL MONITORING AND COMPUTING, SPRINGER NETHERLANDS, DORDRECHT, vol. 31, no. 2, 14 March 2016 (2016-03-14), pages 459 - 467, XP036178350, ISSN: 1387-1307, [retrieved on 20160314], DOI: 10.1007/S10877-016-9862-4

## Description

### FIELD OF INVENTION

The present invention is directed generally to a method and apparatus for non-invasive measurement and monitoring of intraorbital pressure waves.

### BACKGROUND OF THE INVENTION

Traumatic brain injury ("TBI") is a serious problem facing up to 2.5 million people each year worldwide. Presently only 10% of TBI patients survive without serious cognitive problems and 25% of TBP patients die in developed countries as a result of their injuries. The remaining 65% of TBI patients that survive have serious problems with quality-of-life issues. The costs for caring for TBI patients in well developed countries have been estimated to be as high $ 400 billion U.S. dollars annually (HOME/CENTER-TBI: Fact sheets. https://www.center-tbi.eu). Any methods or apparatus that will improve the diagnosis and treatment of these patients will offer great benefits to those patients and society.

According to many TBI treatment professionals, one area of need is simpler and more accurate and higher resolution method for monitoring intracranial pressure (ICP) waves of TBI patients. It would be desirable to have a method of measuring and monitoring ICP pulse waves that is simpler to use and more accurate and less invasive then existing invasive monitoring methods. It would also be desirable to have a method and device for monitoring ICP pulse waves in a single hemisphere of the human intracranial media with higher resolution and precision than existing invasive intracranial parenchymal sensors. Existing invasive methods can be dangerous because they require removing parts of the patient's skull to permit placement of the sensors. Less invasive ICP monitoring methods use external sensors outside the patient's skull and measure across the patient's skull generating measurements with lower resolution, lower precision and lower correlation with the patient's actual ICP. Existing methods to measure ICP pressure dynamics are presently limited to invasive methods or methods that have low measurement resolution and precision. Non-invasive intra-orbital pressure measurements are known from US2018/279877A1.

A desired method of intraorbital pressure dynamics measurement and monitoring would be non-invasive and have higher resolution than invasive ICP(t) monitoring methods for monitoring of the ICP wave morphology changes. ICP morphology changes provide diagnostic information on brain compliance changes and increments of ICP value.

What is needed is a method of non-invasive measurement of the patient's intraorbital pressure that would permit direct monitoring of the patient's ICP waves. What is also needed is a non-invasive method would also be safer for the patient than the known invasive methods thus reducing danger to the TBI patient. It would also be desirable to have a method or device that used a passive sensor that could be easily applied non-invasively to the outside of the patient's eye with relatively little or no pressure (Pe) applied to a closed eye lid of the TBI patient.

### BRIEF SUMMARY OF THE INVENTION

The invention is defined by the appended claims. The inventive method and device measures patient specific ICP(t) pulse waves shape non-invasively with better precision with more clear fronts, peaks and singularity points than invasively measured ICP(t) pulse wave shape in a brain parenchyma. The invention measures an intracranial pressure dynamic in one hemisphere of human brain through one eye. The optic nerve sheath of a TBI patient pulsates in the same way as the cerebral spinal fluid volume pulsates inside optic nerve subarachnoid space. That pulsation (intracranial slow, respiratory and pulse waves) creates micro movement of the patient's eye and the orbital tissues. The innovative device and method uses a noninvasive method and apparatus to measure a ICP(t) pulse wave by measuring intraorbital pressure waves. This inventive method and apparatus is a safer ICP monitoring technique that yields data with higher resolution than existing methods.

The apparatus for non-invasive intracranial wave measurement comprises a sealed rigid pressure applicator filled with degasified water. The pressure applicator includes a water filled bladder with a pressure (Pe) sensor extending inside the bladder. The bladder comprises a thin elastic non-allergenic film filled with an internal media such as water or some other non-compressible liquid. The bladder is positioned on an exterior portion of the pressure applicator. The pressure applicator has a sealing component that seals the applicator with the facial tissues surrounding the patient's eye while at the same time being physically connected with the patient's closed eyelid by the thin elastic non-allergenic film.

In some embodiments the internal media is degasified water but other noncompressible liquids or gels could also be used. The invention includes a pressure sensor extended inside the bladder for measuring the pressure dynamics inside the bladder. A sealing component extends from and connects to the pressure applicator and is configured to hermetically seal the pressure applicator around the patient's eye and orbital area. A micro-pump, or some other type of liquid pump is connected to the bladder to adjust the volume and pressure of the internal media to the bladder. The pump is also connected to the control unit for controlling the volume of internal media inside the bladder. The control unit also includes a display that can be capable of displaying the amount of volume of the media inside the bladder and pressure within the bladder. The volume of the media inside the pad is adjusted by the pump for each individual patient before any of the measurements of ICP(t) pulse waves are made. The volume of media inside the pad is adjusted to ensure full physical contact of the internal liquid media inside the pad with the patient's closed eye lid and all patient's tissues surrounding the eye which are covered by thin elastic film. The volume of the internal media is also adjusted to remove all air from the initial gap between the patient's eye and surrounding facial tissues and the pressure applicator's bladder before the start of any pressure measurements. The control unit to which the pressure sensor is connected is also capable of displaying the measured pressure inside the bladder being measured by the pressure sensor in the bladder. The dynamic pressure measurements are measurements of ICP(t) pulse waves. The optimum maximal internal volume of water inside the bladder can be maintained by using between 1mmHg and 2mmHg of internal pressure inside the bladder during ICP(t) dynamic monitoring. This is a relatively low amount of pressure being applied to the patient's closed eyelid making the application of the bladder to the patient's eye extremely safe when compared to existing invasive methods.

The pressure sensor is configured to measure changes in pressure in the bladder and to send measurement signals to the control unit for display of the measured changes in pressure in wave form. The pressure applicator has a sealing component that seals the applicator with the facial tissues surrounding the patient's eye while at the same time being physically connected with the patient's closed eyelid by the thin elastic non-allergenic film. The Pe(t) dynamic measurements taken by the pressure sensor are then recorded and displayed for the TBI professionals to review a morphology of ICP(t) pulse wave and to interpret it.

Interpretation of ICP(t) pulse wave morphology is related to measurement of such morphological parameters as the peaks' ratios. The peaks' ratios are associated with dangerous patients' secondary brain insults caused by decrements of brain compliance or increments of ICP value.

The inventive method is for a non-invasive measurement and monitoring of intraorbital pressure waves of a patient comprising; placing a pressure applicator with a bladder containing a pressure sensor on a patient's closed eye lid; sealing the pressure applicator to the patient's orbital area; increasing water volume in the bladder to remove any are between the bladder and the patient's orbital area; measuring the changes in the patient's intraorbital pressure on the bladder; displaying changes in the patient's intra orbital pressure in wave form with a control unit connected to the pressure sensor.

The bladder is connected to the water pump to adjust the internal volume and pressure of the water in the bladder. The Pe sensor inside the bladder is connected to a control unit with a display capable of displaying the extraorbital pressure wave measured by the Pe sensor. The measured extraorbital pressure wave correlates directly with the patient's intraorbital pressure waves and also directly correlates with the patient's ICP (t) waves.

### BRIEF DESCRIPTON OF THE FIGURES

Figure 1 is a drawing of one embodiment of an apparatus for measurement of intraoribital pressure waves according to the invention.
Figure 2 is a flow chart and algorithm of one embodiment of the non-invasive intracranial measurement method according to the invention.
Figure 3 is a representation of one embodiment of pressure applicator according to the invention without a sealing component.
Figure 4 is a representation of one embodiment of the pressure applicator with a sealing component positioned on the closed eye of a patient according to the invention.
Figure 5 is a representation of one embodiment of the pressure applicator positioned on the closed eye of a patient and an ultrasonic device used to measure and display a view of the patient's carotid artery.
Figure 6 is a representation of one embodiment of the pressure applicator positioned on the closed eye of a patient and an ultrasonic device used to measure and display a view of the patient's jugular vein.
Figure 7(a) is a graph showing intraocular pressure pulse waves of a healthy patient during a jugular vein compression test.
Figures 7(b) and 7(c) show ultrasound images of a patient's carotid artery and jugular vein during a jugular vein compression test.
Figure 8(a) is a graph showing intraocular pressure pulse waves of a healthy patient during a controlled common carotid artery compression test.
Figures 8(b) and 8(c) show ultrasound images of a patient's carotid artery and jugular vein during a controlled common carotid artery compression test.
Figure 9 is a representation of patient being monitored with invasive ICP pulse wave monitoring device and with non-invasive intraorbital pulse wave monitoring device according to the invention.
Figure 10 is graphs showing the signals of simultaneous invasive ICP(t) wave monitoring and non-invasive intraorbital pressure pulse wave monitoring on a patient.
Figure 11 is graphs showing the signals of simultaneous invasive ICP(t) wave monitoring and non-invasive intraorbital pressure pulse wave monitoring using extraorbital pressure measurements on a patient.
Figure 12 are a graphs showing comparison of normalized intracranial pressure and noninvasive extraorbital pressure waves and normalized wave spectra.
Figure 13 are graphs showing comparison of invasively recorded ICP(t) pulse wave and simultaneously recorded extraorbital pressure pulse wave on a patient.
Figure 14 are graphs showing comparison of invasively recorded ICP(t) pulse wave and simultaneously recorded extraorbital pressure pulse wave on a patient.

### DETAILED DESCRIPTION OF THE INVENTION

The inventors have invented a new device and method recognizing that human eye intraorbital pressure waves reflect ICP pulse waves in a hemisphere of the human intracranial media. The invention has higher resolution and precision than existing prior art methods such as invasive intracranial parenchymal pressure sensors and noninvasive external measurement devices and techniques. Existing prior art methods used to measure ICP pulse waves and intraorbital pressure dynamics use either invasive methods that are relatively more dangerous to the patient or noninvasive methods that are less precise methods that measure ICP pulse waves with lower resolution and precision.

The inventors have invented a new device and method of non-invasive measurement using the patient's intraorbital pressure wave which permits direct monitoring of the patient's ICP waves. The invention is a passive and noninvasive device and an improvement over the prior art. Not only does the invention not require sensors be placed inside the skull of the patient, it also does not apply pressure or deformation to the patient's eyeball and does not require transmitting ultrasound, light or radiation into the patient's eye or eye orbit.

The pressure applicator of the invention is a passive and noninvasive device that can operate with applying only a minimum or almost no pressure to the orbit of the patient's eye. The pressure applicator precisely measures the vibration of a patient's eyeball caused by pulsation of their orbital tissues. The pulsation of the orbital tissues is caused by cerebrospinal fluid pulsation in the patient's optic nerve subarachnoid space. The optic nerve sheath of a TBI patient pulsates in the same way as the cerebral spinal fluid volume pulsates inside the optic nerve subarachnoid space. That pulsation (intracranial slow, respiratory and pulse waves) creates micro movement of the patient's eye and the orbital tissues surrounding the optic nerve. Such pulsation is caused first by pulsation and vibration of the optic nerve sheath and it reflects and correlates with all the intracranial pressure ICP(t) waves and changes. The monitor of the control unit records the output signal of the pressure sensor/transducer which is inserted into the bladder, a water filled chamber, part of the pressure applicator. Recorded when practicing the invention, the shape of the recorded output signal measuring extraorbital pressure waves, even without filtering or other signal analysis procedures, has an extremely high correlation with invasive ICP(t) wave signal.

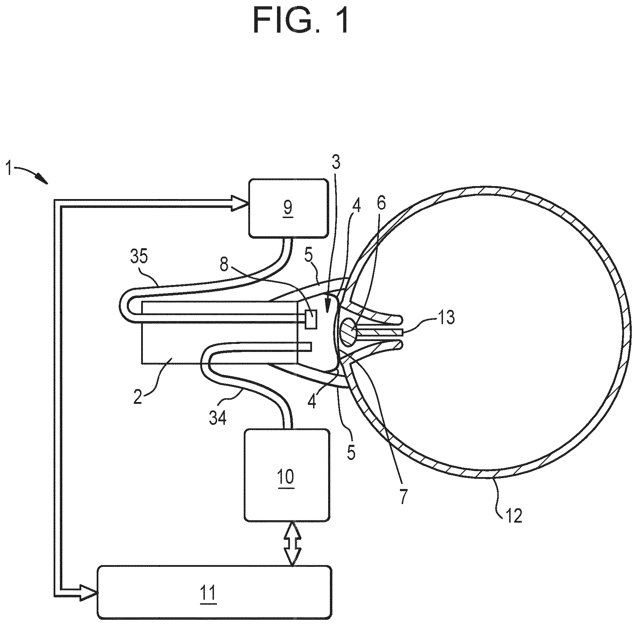

FIG. 1. Shows a diagram of the components for the of apparatus for the non-invasive intracranial waves measurement device 1, which includes a rigid pressure applicator 2 with a water chamber or inflatable bladder 3 formed by a thin elastic nonallergenic film 4 filled with media such as water. The water (having a compliance value close to zero) acts as a receiver of micro movements of the patient's eye. Such micro movement is transferred into electric signal by pressure Pe sensor inside the water pad and in physical contact with the patients closed eyelid. The apparatus also includes a seal 5. The seal 5 is used to substantially hermitically seal the pressure applicator 2 with the tissues surrounding the patient's eye 6 and the pressure applicator 2 is physically connected with the patient's closed eye lid 7 by the thin elastic non-allergic film 4 of the bladder 3. Inside the bladder 3 is a pressure sensor 8 which is immersed in the water inside the bladder 3 and is attached to a pressure meter 9 for recording and saving pressure measurements. The pressure meter 9 is also connected to the control/display unit 11. The control/display unit can send and receive instructions and information from both the pressure meter 9 and/or micro water pump 10 for controlling those devices and for saving and displaying information received from those devices. Embodiments of the invention could include "blue tooth" or other wireless communication between the pressure sensor and a receiver in an outside device such as a mobile phone or control unit/display 11 that can be used to control the pressure applicator and transmit other data or information from the sensor. The pressure meter 9 can be connected to the control unit 11 for controlling the pressure meter and recording and saving measured results and displaying the measured results..

The sealing of the pressure applicator 2 to the facial tissues, facial bones and skull 12 surrounding the patient's eye creates a closed rigid box with close to zero internal compliance when applied to and eye and surrounding tissues. After initial sealing, the volume of water inside the bladder 3 is adjusted by a micro or water pump 10 before starting measurements. The water pump 10 is needed to ensure full mechanical contact between the bladder 3 and the patient's closed eyelid 7 and surrounding structures of the patient's face after hermitically sealing of the pressure applicator 2 to the face of the patient. The water pump 10 is connected to the control unit 11 which activates the pump 10 to incrementally increase the water inside the bladder3. The control unit 11 can also display information related to the pump 10. The water volume is controlled by the the control unit 11 which gradually increases in the bladder 3 in order to remove all air from the gap between elastic film and the patient's closed eye lid 7 and tissue surrounding the patient's eye and in order to achieve close to zero compliance of internal media of pressure applicator 2. Measurement of the intraorbital pressure waves, resulting from movement of the patient's eye 6 and orbital tissue surrounding the optic nerve 13, does not start until the volume of water inside a water pad is maximal in order to get full physical contact between the thin film 4 of the bladder 3 and the eyelid 7 and surrounding tissues of an individual patient. Minimal pressure between 0-2 mmHg is sufficient to remove all the air beneath the sealed area. Typically, a pressure of just 1 mmHg or less of measured pressure inside the water is sufficient to remove all the air from any internal gap between the patient's eyelid 7 and the elastic film 4 of the bladder and substantially all the air beneath the seal.

The intraorbital pulse wave pressure is measured by a pressure meter 9 with a pressure sensor 8 that extends into the bladder 3. The pressure sensor 8 has a pressure resolution less than 0.01 mmHg because the device needs to measure pulse waves that have an amplitude of approximately 1.0-2.0 mmHg but sometimes even less than 1.0 mmHg.

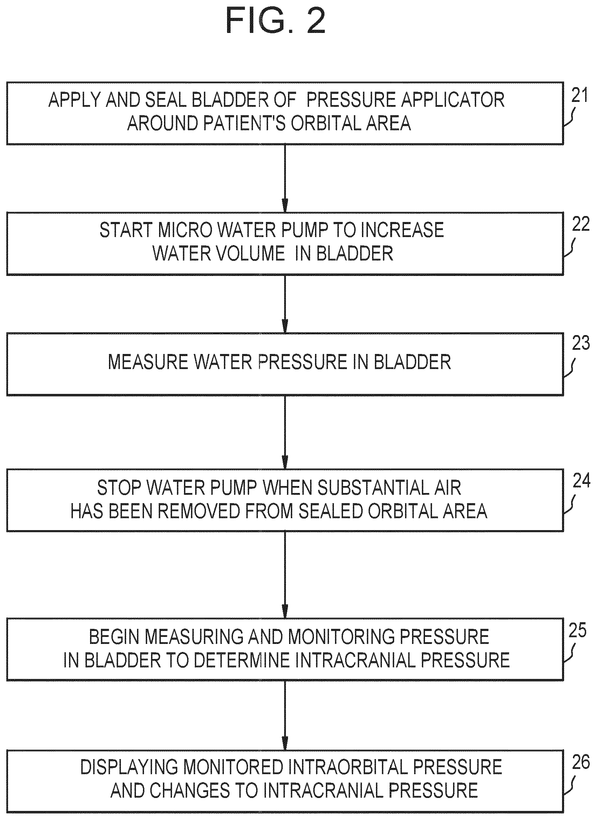

FIG. 2. Shows the algorithm of the non-invasive intracranial wave measurement process. First, the bladder 3 of the pressure applicator is applied to the patient's orbital area and sealed on the closed eyelid 7 of the patient 21. Next the micro pump is started and with a deliberate and controlled step by step process of increasing the water volume in the bladder 22. The water pressure in the bladder 3 is measured to be sure it does not increase beyond 2mmHg 23. The water pump 10 is stopped when substantially all the air has been removed from beneath the seal 5 on the patient's orbital area 24. This should occur before the pressure reaches 2mmHg. Once the needed maximal volume of water in the bladder 3 is achieved and the water pump 10 is stopped the measuring and monitoring of pressure begins to determine intraorbital and intracranial pressure 25. Monitoring of the pressure waves Pe(t) begins after micro volume pump 10 stops. The measured Pe(t) wave shape is then displayed on the display monitor 11 and shows wave morphology and any other changes to the measured waves. The display 11 then displays the monitored intraorbital pressure and changes to the intracranial pressure so health care professionals can monitor changes to ICP(t) and take appropriate health care actions for the TBI patient. 26

This method could also be followed using two devices one on each of the patient's eyes allows one to measure ICP(t) dynamics in the two hemispheres of the patient's brain simultaneously. This permits comparison of the ICP dynamics in a patient's healthy hemisphere with the patient's injured hemisphere. Measuring ICP in two hemisphere's of the patient is impossible using invasive ICP(t) measurements because it is dangerous and unethical to implant invasive ICP sensor into the injured hemisphere of brain together with an other ICP sensor in healthy hemisphere of the brain. The invention is an advancement in neurosurgical intensive care of traumatic brain injury patients and neurosurgical patients.

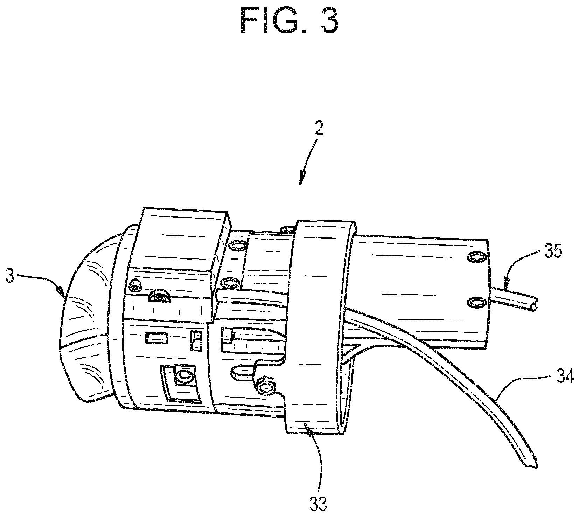

FIG. 3. Shows a representation of one embodiment of a pressure applicator 2 without a sealing component. The pressure applicator comprises a bladder 2 and a gripping area 33. The pressure applicator also includes a flexible water connector 34 for connecting the bladder 3 to the water pump 10 and a sensor connector 35 that connects the pressure sensor 8 to the pressure meter 9. The water connectors 34 remain full of water during intraorbital/intracranial pressure monitoring. Two pressure applicators can be used individually on both eyes of a patient simultaneously. This use of dual applications can be used for improved diagnosis and also presents opportunity for glaucoma or brain injury diagnostics and treatment monitoring. The pressure sensor 8 is located inside the water chamber 3 or bladder of the applicator and it is immersed into water. The pressure sensor 8 is connected to a control/display unit 11 with a display for recording and displaying the measured Pe(t) waves.

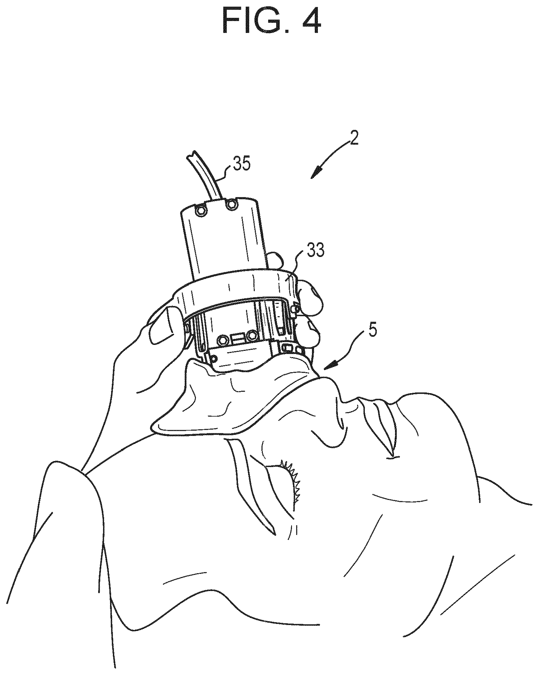

FIG. 4. Shows a pressure applicator 2 with a seal 5 gently resting on the eye orbit of a patient. The seal or sealing component 5, is used to hermetically seal the pressure applicator 2 to the eye orbit of the patient. One embodiment of a pressure applicator 2 according to the invention uses medical plastic as a seal 5 to seal the pressure applicator 2 to the patient's eye orbit. Another example of soft materials that can be used as a sealing component include a vinyl polysiloxane (VPS) used for medical dental impressions. Other gentle flexible and adaptive mechanical sealing material or other methods can be used for sealing the pressure applicator 2 to the patient's eye orbit. Other embodiments of the pressure applicator 2 could use different sealing components as such as a solid flexible flap similar to those used with plastic swimming goggles. Other gentle mechanical sealing components could also be used. While the seal does not need to be completely 100 % hermetically sealed to the patients face (meaning absolutely no air remains under the seal after the water volume in the bladder pressure is adjusted) the hermetic seal does need to sufficiently seal to the patients face in a way that allows substantially all the air pockets beneath the seal 5 to be removed while preventing reentry of air beneath the seal 5 during monitoring. The seal 5 should maintain the rigid box structure of the pressure applicator to allow for accurate measurements. The air pockets beneath the seal need to be minimized to make accurate pressure measurements. One embodiment could include a oneway seal that allows air to escape during the increase in water pressure but prevents the escaped air from reentering below the seal after the water pressure stops increasing.

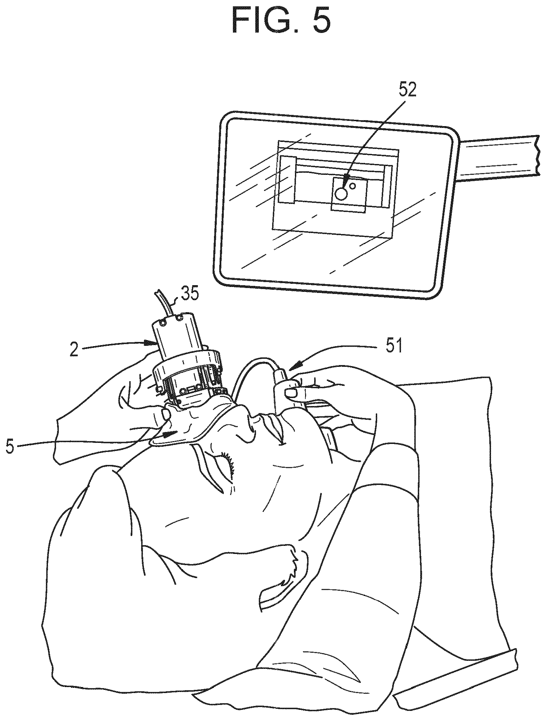

FIG. 5 Shows a representation of one embodiment of the pressure applicator 2 on the eye orbit and closed eye of a patient with an ultrasonic device 51 used to measure and display a view of the patient's carotid artery 52. The ultrasonic view screen of the patient's carotid artery 52 is examined by a triplex ultrasonic scanner. During testing an Epiq Elite (Philips) ultrasound device was used.

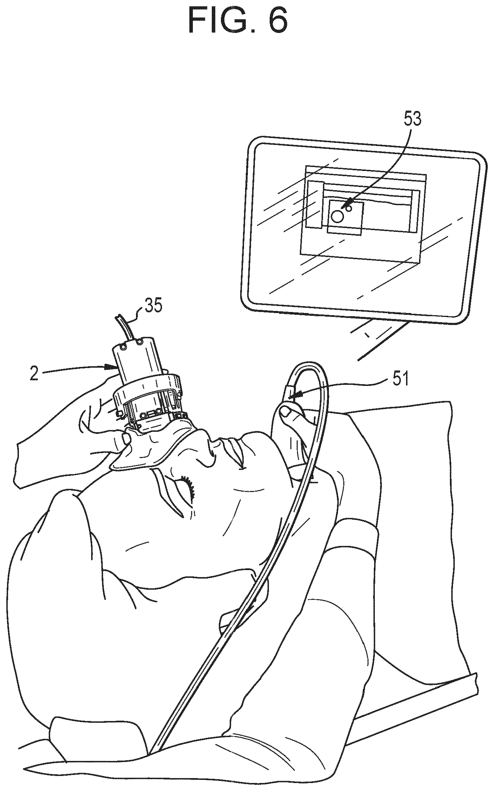

FIG. 6. Shows a representation of one embodiment of the pressure applicator 2 positioned on the eye orbit and closed eye of a patient and an ultrasonic device 51 used to measure and display a view of the patient's jugular vein. 53 The ultrasonic view of the patient's jugular vein 53 is examined by triplex ultrasonic scanner. During testing an Epiq Elite (Philips) ultrasound device was used.

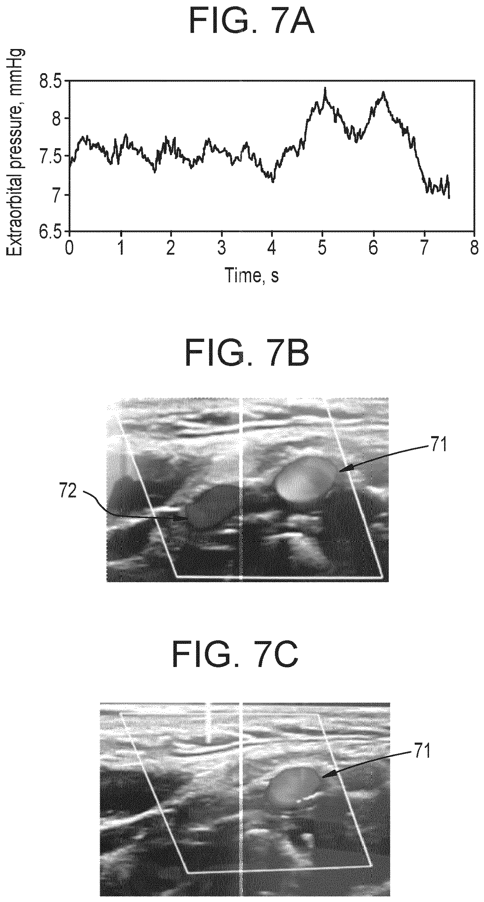

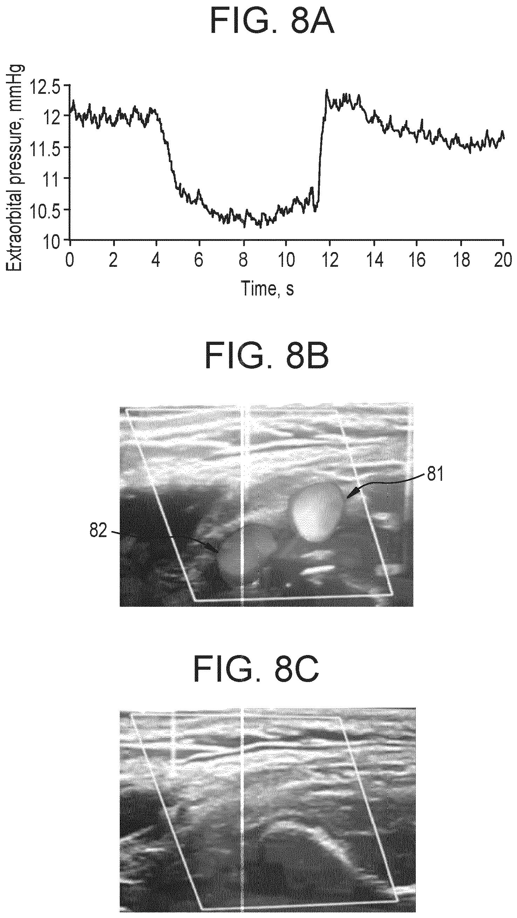

Fig. 7(a). is a graph showing extraorbital pressure waves measurement of the intraorbital pulse waves of a healthy volunteer during a jugular vein compression test. Philips EPIQ Elite equipment was used to conduct the controlled jugular vein compression test of healthy volunteer in a supine position. Both external jugular veins were gently compressed two times starting from 4 sec. and finishing at 6.3 sec. Fig. 7(b) and Fig. 7(c) show ultrasound images of the patient's carotid artery 71 and jugular vein 72 during the jugular vein compression test. The initial scan of blood flow Fig. 7(b) in the jugular vein 72 and common carotid artery 71 shows normal blood flows. The full jugular veins compression Fig. 8(c) shows only carotid artery flow 71 and no venous flow.

The jugular vein compression test with the healthy volunteer in a supine body position increases intracranial blood volume and intracranial blood pressure at two points during the measurement and are clearly seen in the Fig. 7(a) extraorbital pressure wave measurements at approximately 5.1 sec and 6.2 sec.

The cause of the peaks of the extraorbital and measured intraorbital pressure waves is ICP(t) waves and cerebrospinal fluid pulsation inside of optic nerve canal's subarachnoid space. Fig 7 shows raw extraorbital pressure pulse waves measurement (no filtering of noises) and reactions of intraorbital pressure to physiological tests which increases ICP or decreases ICP.

FIG. 8(a) is a graph showing intraorbital pulse waves of a healthy volunteer during a controlled common carotid artery compression test. The test is of a healthy volunteer in supine position. One common carotid artery was compressed starting from second 4 and released at 11.6 second (Fig. 8 a). FIGs. 8(b) and 8(c) show ultrasound images of thr patient's carotid artery 81 and jugular vein 82 during the jugular vein compression test.

Initial blood flow is shown in the ultrasound image of FIG. 8(b) in the jugular vein 87 and common carotid artery 82 show normal blood flows. The ultrasound image of FIG. 8 (c) shows no venous or arterial flow after compression of the jugular vein and carotid artery.

The common carotid artery compression in supine body position decreases intracranial blood volume and intracranial blood pressure. Decrement in ICP(t) is clearly seen in Fig.8(a) starting at approximately 4.5 sec. while release of the compressed artery at 11.6 second provokes an exponential transient reaction (transient function) of cerebral blood flow autoregulation system with typical seven second duration for a healthy volunteer. Recorded reactions in FIGS. 7 and 8 of the pressure inside of pressure applicator is evidence that the proposed apparatus is directly monitoring an ICP changes.

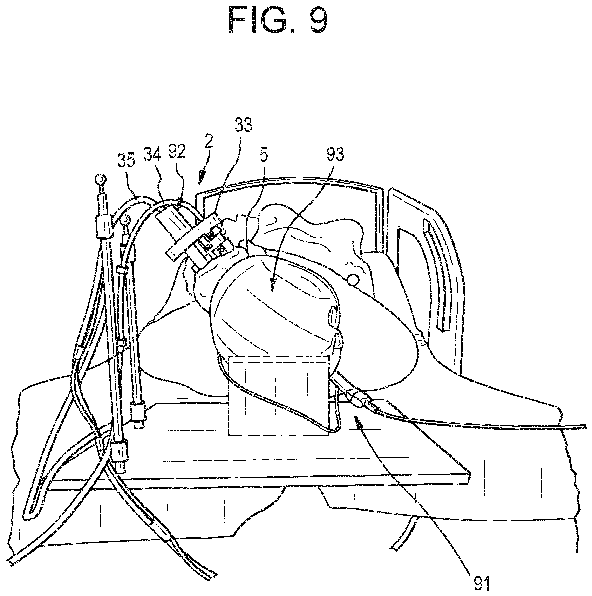

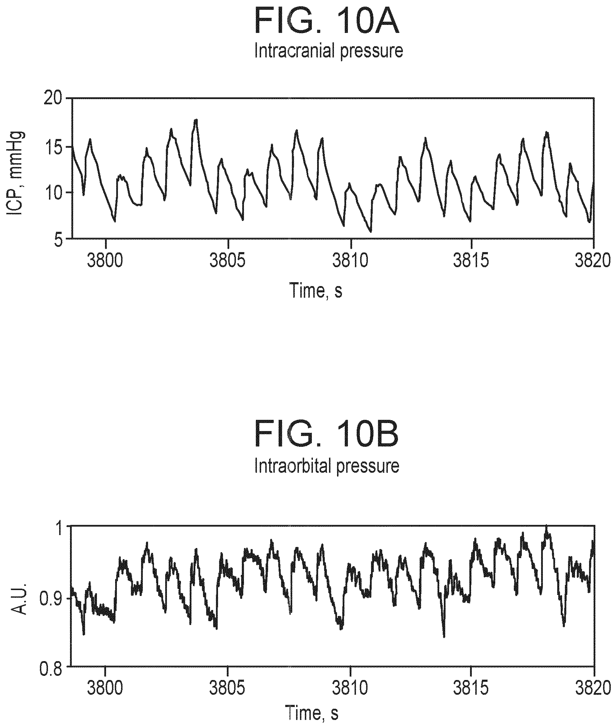

FIG. 9 shows a representation of patient being monitored with invasive ICP pulse wave monitoring equipment 91 and with the non-invasive intraorbital pulse wave monitoring device 92 according to the invention. The figure shows the head of a TBI patient 93 in a supine position with an invasive ICP pulse wave monitoring device 91 with the inventive noninvasive intraorbital pulse wave monitoring device 92. The invasive ICP pulse wave test monitoring of a patient takes place at the same time as the noninvasive intraorbital pressure wave monitor using the inventive device and the resulting measured pulse waves shows a very high level of correlation between the two measurement methods as can be seen in Figures 10 -14.

Figure 10 is graphs of measurement data of simultaneous monitoring of intracranial pressure using a parenhymal ICP(t) transducer, a Codman ICP EXPRESS monitor and a Draeger Infinity Delta monitor shown in Fig. 10(a) and noninvasive intraorbital pressure wave raw signals shown in Fig. 10(b). The figure shows ICP measurements in mmHg and arbitrary units and shows time in seconds. Those of skill in the art would recognize other sensors or monitors could be used to measure ICP pulse waves. The graphs demonstrate the correlation between the two pulse wave measurements is direct and very high with a correlation factor R = 0.99. The correlation factor of 0.99 is achieved comparing the synchronous recordings of a single (snapshot measurement of the on pulse wave) noninvasive pulse wave using the noninvasive invention with a single invasively measured pulse wave. A correlation factor R = 0.91 is achieved in the case of invasive and noninvasive pulse wave continuous monitoring, when comparing the continuous monitoring data. Monitoring means repeatable measurements, over an extended period of time, of pulse waves which are modulated by the respiration process and influenced by other factors including artifacts which decrease the maximum possible correlation between monitored time series data. Diagnostic information is in different ratios of pulse wave peaks, fronts and singularity points of the pulse wave shape. The ICP wave shape is presented in arbitrary units (AU) for morphological analysis of a wave shape because the arbitrary units (ratios of amplitudes of pulse wave peaks) or time units (durations of pulse wave fronts or distances in time scale) are used for analysis of pulse wave morphology and TBI patients' treatment decision making.

Figures 10(a) and 10(b) are graphs of the raw signals of invasively monitoring ICP(t) waves measured simultaneously with non-invasively monitoring intraorbital pressure pulse waves of a patient with severe TBI. Pulse waves (period approximately 1 second) and respiratory waves (period approximately 5 seconds) are clearly seen in both graphs.

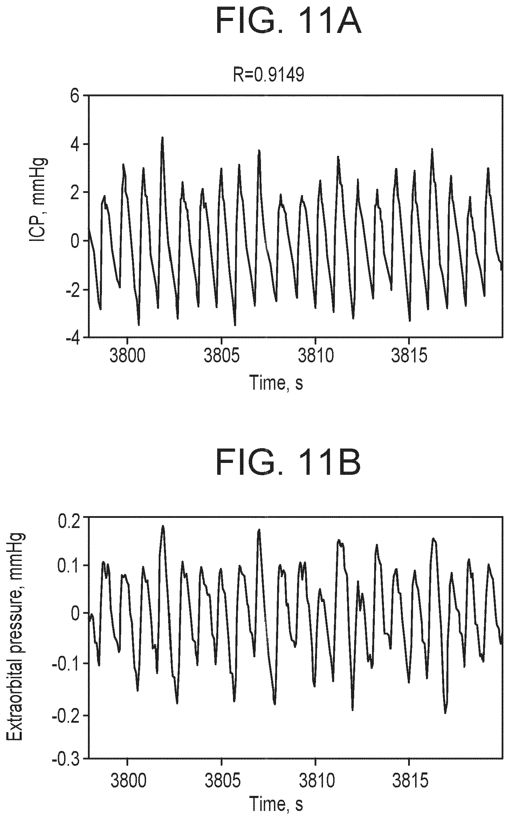

Figure 11 is another example of graphs showing the simultaneous monitoring a patient's ICP pulse waves using an invasive method while monitoring the patient's intraorbital pulse waves using the inventive noninvasive method. Fig 11(a) shows the results of invasive measurement of ICP pulse waves using a Codman invasive ICP sensor and monitor. The figure shows ICP measurements and extraorbital pressure measurements in mmHg and shows time in seconds Those of skill in the art would recognize other sensors or monitors could be used to measure ICP pulse waves. Fig. 11(b) shows the simultaneously measured results from non-invasive intraorbital pressure pulse wave monitoring using the inventive device and method to measure extraoribital pressure. The correlation between the simultaneously recorded data sets recordings is above R=0.91. The noninvasively recorded intraorbital pressure pulse waves show more clearly defined physiological peaks, singularity points and morphology when compared with the invasively recorded ICP pulse waves.

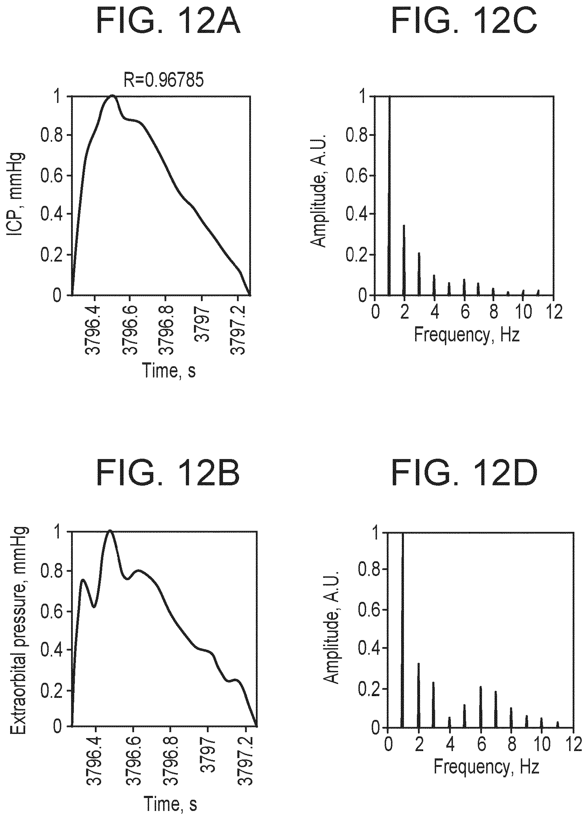

Figure 12 are graphs showing a comparison of normalized invasive intracranial pressure and noninvasive extraorbital pressure waves and normalized spectra of waves calculated by Fourier spectral analysis. Fig 12(a) and (b) show ICP measurements and extraorbital pressure measurements in mmHg and shows time in seconds. Figures 12(c) and (d) show amplitude in arbitrary units and frequency in Hz. It is seen that resolution of non-invasive monitoring of pulse waves in graph 12b is higher compared with invasive parenchymal ICP(t) pulse and respiratory wave monitoring in graph 12a. The three peaks and other morphological parameters are better expressed in the noninvasively recorded pulse wave graph 12b. The graphs show that the noninvasively recorded extraorbital pulse wave spectrum harmonics 12d are relatively higher when compared with the invasive parenhymal ICP(t) pulse wave spectrum harmonics 12c in the spectrum interval above 4^{th} upper harmonic of ICP(t) pulse wave. This is an evidence that extraorbital pressure pulse waves (which reflect intraorbital pulse waves) 12b provide diagnostic information on intracranial pressure dynamics in a brain's hemisphere with higher precision compared with invasive parenchymal pressure measurements.

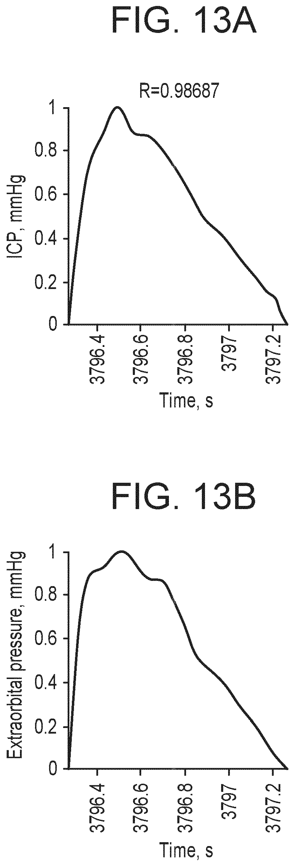

Figure 13 are graph comparisons of invasively recorded ICP(t) pulse waves and simultaneously recorded extraorbital pressure pulse waves from the same patient. Figures 13(a) and (b) show graph comparisons of invasively recorded ICP(t) pulse wave on the same patient and simultaneously recorded extraorbital pressure pulse wave. Correlation factor R between both measured pressure waves is approximately R=99 (R=0.98687). The high correlation is an evidence that intraorbital pulse wave is caused by the ICP(t) pulse wave. The calculated correlation between both waves is approximately R=0.99.

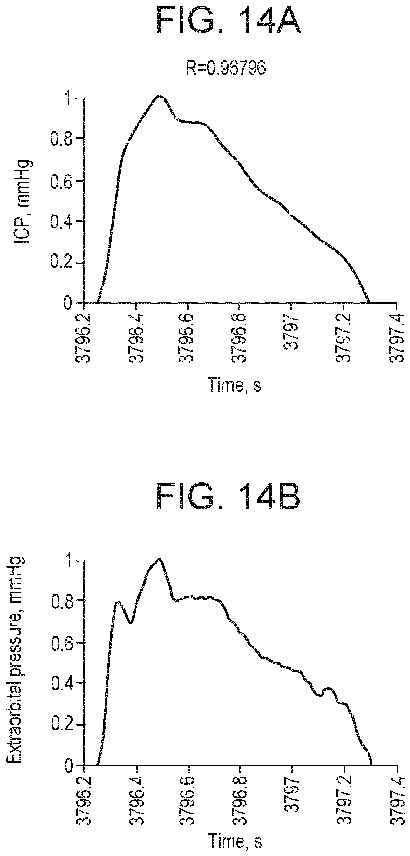

Figure 14 are also graphs showing comparison of invasively recorded ICP(t) pulse wave and simultaneously recorded extraorbital pressure pulse wave on a patient. It is seen on Figure 14 that morphology of ICP(t) pulse wave is expressed much more clearly by non-invasively recorded intraorbital pulse waves. The figure shows the comparison of simultaneously recorded normalized invasive parenhymal intracranial pressure pulse wave Fig. 14(a) and non-invasive extraorbital pressure pulse wave Fig. 14(b) This figure is also evidence of the causality that the cause of the noninvasively recorded pulse wave is the ICP(t) pulse wave. The identified non-invasively recorded intraorbital pressure pulse wave 14b has much clearly expressed physiological peaks, singularity points and morphology of shape comparing with-invasively recorded pulse wave in 14a. The correlation between the intraorbital pulse wave and the ICP(t) pulse wave is R= 0.96796. This lower correlation is used in order to record better expressed peaks and other morphological parameters of the noninvasively measured pulse wave.

## Claims

1. An apparatus for non-invasive measurement and monitoring of intraorbital pressure waves of a patient comprising:
a pressure applicator (2) with a bladder (3);
the bladder comprising an elastic film (4) filled with a non-compressible media, positioned on the exterior of the pressure applicator;
a pressure sensor (8) inside the bladder connected to a pressure meter (9);
a sealing component (5) extending and connected to the pressure applicator configured to hermetically seal the pressure applicator around the patient's eye (6);
a pump (10) connected to the bladder to control and adjust the volume of the media to the bladder;
the pump also being connected to a control unit (11) for controlling the volume and pressure of media in the bladder;
the pressure sensor being configured to measure changes in pressure in the bladder and to send measurement signals to the pressure meter;
the pressure meter being connected the control unit and being configured to transmit data and pressure measurement data to the control unit;
the control unit being configured to record and store the pressure measurement data; and
a display connected to the control unit configured to display the pressure measurement data.

2. The apparatus of claim 1 where the media is water and the pump is a water pump.

3. The apparatus of claim 2 where the pressure measurement data is displayed in wave form.

4. The apparatus of claim 3 where in the wave form displayed is the extracranial orbital pressure wave of a patient.

5. The apparatus of claim 4 where the wave form displayed is the intra-cranial pressure, ICP, brain wave of a patient.

6. A method for non-invasive measurement and monitoring of intraorbital pressure waves of a patient comprising;
applying a pressure applicator of claim 1 comprising a pressure sensor in a bladder filled with a liquid medium to the closed eyelid of a patient;
sealing the bladder of the pressure applicator to a patient's orbital area;
pumping water into the bladder to increase the water volume in the bladder measuring the medium pressure in the bladder increasing the water volume in the bladder until all the air is forced out from beneath the seal and the pressure applicator is hermetically sealed to the patient's orbital area; measuring changes in the medium pressure in the bladder;
recording and displaying pressure changes in wave form with a control unit connected to the pressure sensor.

7. The method of claim 6 where the media is water.

8. The method of claim 6 where the pressure measurement data is recorded for at least 20 seconds.

9. The method of claim 6 where a second pressure applicator is applied to the patient's second orbital area.

## Patentansprüche

1. Vorrichtung zur nicht-invasiven Messung und Überwachung von intraorbitalen Druckwellen eines Patienten, umfassend:
einen Druckapplikator (2) mit einer Blase (3);
wobei die Blase einen elastischen Film (4) umfasst, die mit einem nicht komprimierbaren Medium gefüllt ist, die auf der Außenseite des Druckapplikators positioniert ist;
einen Drucksensor (8) im Inneren der Blase, der an einen Druckmesser (9) angeschlossen ist;
eine Dichtungskomponente (5), die sich zu dem Druckapplikator erstreckt und an diesen angeschlossen ist, die dazu konfiguriert ist, den Druckapplikator um das Auge (6) des Patienten herum hermetisch abzudichten;
eine Pumpe (10), die an die Blase angeschlossen ist, um das Volumen des der Blase zugeführten Mediums zu steuern und einzustellen;
wobei die Pumpe auch an eine Steuereinheit (11) zum Steuern des Volumens und des Drucks von Medien in der Blase angeschlossen ist;
wobei der Drucksensor dazu konfiguriert ist, Druckänderungen in der Blase zu messen und Messsignale an den Druckmesser zu senden;
wobei der Druckmesser an die Steuereinheit angeschlossen und dazu konfiguriert ist, Daten und Druckmessdaten an die Steuereinheit zu übertragen;
wobei die Steuereinheit dazu konfiguriert ist, die Druckmessdaten aufzuzeichnen und zu speichern; und
eine Anzeige, die an die Steuereinheit angeschlossen ist, die dazu konfiguriert, die Druckmessdaten anzuzeigen.

2. Vorrichtung nach Anspruch 1, wobei das Medium Wasser ist und die Pumpe eine Wasserpumpe ist.

3. Vorrichtung nach Anspruch 2, wobei die Druckmessdaten in Wellenform angezeigt werden.

4. Vorrichtung nach Anspruch 3, wobei die angezeigte Wellenform die extrakranielle orbitale Druckwelle eines Patienten ist.

5. Vorrichtung nach Anspruch 4, wobei die angezeigte Wellenform die intrakranielle Druck-, ICP-, Gehirnwelle eines Patienten ist.

6. Verfahren zur nicht-invasiven Messung und Überwachung von intraorbitalen Druckwellen eines Patienten, umfassend:
Anwenden eines Druckapplikators nach Anspruch 1, umfassend einen Drucksensor in einer Blase, die mit einem flüssigen Medium gefüllt ist, auf das geschlossene Augenlid eines Patienten;
Abdichten der Blase des Druckapplikators an dem Orbitalbereich eines Patienten;
Pumpen von Wasser in die Blase, um das Wasservolumen in der Blase zu erhöhen
Messen des Mediendrucks in der Blase
Erhöhen des Wasservolumens in der Blase, bis die gesamte Luft unterhalb der Dichtung herausgezwungen wird und der Druckapplikator hermetisch an dem Orbitalbereich des Patienten abgedichtet ist;
Messen von Änderungen des Mediendrucks in der Blase;
Aufzeichnen und Anzeigen von Druckänderungen der Wellenform mit einer an den Drucksensor angeschlossenen Steuereinheit.

7. Verfahren nach Anspruch 6, wobei das Medium Wasser ist.

8. Verfahren nach Anspruch 6, wobei die Druckmessdaten für mindestens 20 Sekunden aufgezeichnet werden.

9. Verfahren nach Anspruch 6, wobei ein zweiter Druckapplikator auf den zweiten Orbitalbereich des Patienten angewendet wird.

## Revendications

1. Appareil de mesure et de surveillance non invasives des ondes de pression intra-orbitales d'un patient, comprenant :
un applicateur de pression (2) muni d'une vessie (3) ;
la vessie comprenant un film élastique (4) rempli d'un milieu incompressible, positionné à l'extérieur de l'applicateur de pression ;
un capteur de pression (8) à l'intérieur de la vessie, relié à un manomètre (9) ;
un élément d'étanchéité (5) s'étendant depuis l'applicateur de pression et relié à celui-ci, configuré pour sceller hermétiquement l'applicateur de pression autour de l'œil (6) du patient ;
une pompe (10) reliée à la vessie pour contrôler et ajuster le volume du milieu dans la vessie ;
la pompe étant également reliée à une unité de commande (11) pour contrôler le volume et la pression du milieu dans la vessie ;
le capteur de pression étant configuré pour mesurer les variations de pression dans la vessie et pour envoyer des signaux de mesure au manomètre ;
le manomètre étant relié à l'unité de commande et étant configuré pour transmettre des données et des données de mesure de pression à l'unité de commande ;
l'unité de commande étant configurée pour enregistrer et stocker les données de mesure de pression ; et
un écran relié à l'unité de commande et configuré pour afficher les données de mesure de pression.

2. Appareil selon la revendication 1, dans lequel le milieu est de l'eau et la pompe est une pompe à eau.

3. Appareil selon la revendication 2, dans lequel les données de mesure de pression sont affichées sous forme d'onde.

4. Appareil selon la revendication 3, dans lequel la forme d'onde affichée est l'onde de pression orbitale extracrânienne d'un patient.

5. Appareil selon la revendication 4, dans lequel la forme d'onde affichée est l'onde de pression intracrânienne (ICP) et cérébrale d'un patient.

6. Procédé de mesure et de surveillance non invasives des ondes de pression intra-orbitales d'un patient, comprenant :
l'application d'un applicateur de pression selon la revendication 1, comprenant un capteur de pression dans une vessie remplie d'un milieu liquide, sur la paupière fermée d'un patient ;
le scellage de la vessie de l'applicateur de pression sur une région orbitale d'un patient
le pompage d'eau dans la vessie pour augmenter le volume d'eau dans la vessie la mesure de la pression du milieu dans la vessie
l'augmentation du le volume d'eau dans la vessie jusqu'à ce que tout l'air soit expulsé de sous le joint et que l'applicateur de pression soit hermétiquement scellé sur la région orbitale du patient ;
la mesure des variations de la pression du milieu dans la vessie ;
enregistrer et afficher les variations de pression sous forme d'onde à l'aide d'une unité de commande connectée au capteur de pression.

7. Procédé selon la revendication 6, dans lequel le milieu est de l'eau.

8. Procédé selon la revendication 6, dans lequel les données de mesure de pression sont enregistrées pendant au moins 20 secondes.

9. Procédé selon la revendication 6, dans lequel un deuxième applicateur de pression est appliqué sur la deuxième zone orbitale du patient.
